(19) Europäisches Patentamt
European Patent Office
Office européen des brevets

(11) Publication number: **0 189 738 B1**

(12) **EUROPEAN PATENT SPECIFICATION**

(45) Date of publication of patent specification: **30.12.92**

(51) Int. Cl.5: **A61K 7/48**, A61K 31/565

(21) Application number: **85850027.5**

(22) Date of filing: **28.01.85**

(54) **Topical compositions for preventing or treating dry skin.**

(43) Date of publication of application:
**06.08.86 Bulletin 86/32**

(45) Publication of the grant of the patent:
**30.12.92 Bulletin 92/53**

(84) Designated Contracting States:
**AT BE CH DE FR GB IT LI LU NL SE**

(56) References cited:
**EP-A- 0 134 954**
**FR-A- 2 405 069**
**US-A- 3 917 829**

**Chemisches Zentralblatt, no. 23, June 8, 1960, p. 7708; V. Facchini: "Erfahrungen über die Einarbeitung neuerer synthetischer Hormone in kosmetische Präparate**

**Chemical Abstracts, vol. 70, 1969, p. 58, ref.no. 34776t; Columbus, Ohio, US P.E. POCHI et al.:"Sebaceous gland response in man to the administration of testosterone, androst-4-enedione, and dehydroisoandrosterone"**

**Chemical Abstracts. vol. 94, 1981, p. 105, ref.no. 77274r; Columbus, Ohio, US H. Schaefer et al.: "Kinetics of percutaneous absorption of steroids"**

**Chemical Abstracts, vol. 99, no. 10, September 1983, p. 370, ref.no. 76899q; Columbus, Ohio, US**

(73) Proprietor: **Orentreich, Norman**
**909 Fifth Avenue**
**New York New York 10021(US)**

(72) Inventor: **Orentreich, Norman**
**909 Fifth Avenue**
**New York New York 10021(US)**

(74) Representative: **Bergvall, Stina-Lena et al**
**Dr. Ludwig Brann Patentbyra AB P.O. Box 17192**
**S-104 62 Stockholm(SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 189 738 B1

## Description

The present invention relates the use of dehydroepiandrosterone and/or a pharmaceutically effective derivative thereof for the manufacture of a medicament for treating dry skin by topical administration of the medicament.

Almost all menopausal and post-menopausal women, many women over thirty-five, and most women over forty years of age, and some older men frequently complain that the natural oiliness of their skin is markedly diminished.

A paper entitled "The Effect of Aging on the Activity of the Sebaceous Gland in Man" by Pochi. P.E. and Strauss, J.S. that was published in Advances in Biology of Skin, Vol VI: Aging, edited by Montagna, W., Pergamon Press, N.Y. (1965), described the reduction in mean sebum (oil) secretion in males and females in relation to advancing age. Figure 1 herein illustrates this reduction in mean sebum production with aging.

Testosterone therapy increases skin oil production in menopausal and post-menopausal women. However, it produces unwanted superfluous facial and body hair and other systemic masculinizing side effects and is therefore rarely used.

Dehydroepiandrosterone (DHEA) is a steroid. It and its sulfate are secreted by the adrenal glands, circulate in the bloodstream, and are excreted in urine as derivatives of DHEA.

As shown in Figure 2, dehydroepiandrosterone sulfate levels in the blood have been shown to reach a peak in early adult life and then gradually decline with advancing age (Orentreich Foundation for the Advancement of Science, Inc., Annual Report, 1979).

The structure for DHEA is as follows:

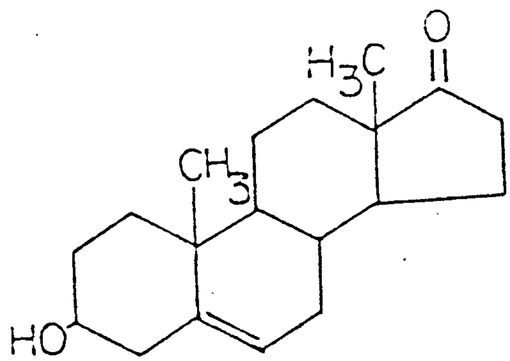

In a study entitled "Biological Activity of Dehydroepiandrosterone Sulfate in Man" by Drucker, W.D., Blumberg, A.M., Gandy, H.M., David, R.R., and Verde, A.L., that was published in the Journal of Clinical Endocrinology and Metabolism, 35: 48-54 (1972), sebum production was used as a measure of androgenic activity associated with the oral administration of dehydroepiandrosterone sulfate. Drucker et al., however, did not address the problem of dry skin in normal older women and some older men. Indeed, the experiments of Drucker et al. were conducted with five abnormal, androgen-deficient, hypogonadal males, ages 15, 20, 30, 34, and 35, and a three-month-old female with 21-trisomy syndrome.

In U.S. Patent 4,005,200, a new use of dehydroepiandrosterone sulfate as a parturient canal conditioning agent was disclosed. Patent 4,005,200 describes systemic administration of dehydroepiandrosterone sulfate to a pregnant female during the 37th to 39th week of pregnancy to improve the maturity of the parturient canal and the sensibility of the uterine musculature to oxytocin. Patent 4,005,200 mentions that it had been proposed to use dehyroepiandrosterone sulfate clinically in combination with estrogens in the treatment of various syndromes associated with climacterium, but the problem of skin dryness was not addressed in this patent.

In recent articles (January 17, 1982 edition of Science News, "Antiobesity Drug May Counter Cancer, Aging"; January 22, 1981 edition of the Chicago Tribune, "Amazing New Drug That May Lead to Longer Life", Kotulak, R.), uses of DHEA and analogs thereof were described to counter obesity and prevent cancer. These articles did not, however, address the problem of skin dryness.

I previously discovered a method for treating dry skin in humans by internally administering an effective dosage of the alcohol and/or one or more salts of DHEA. This treatment is particularly useful in treating dry skin in menopausal women. This treatment can also be employed to treat premenopausal females with a low endogenous DHEA production. Furthermore, males who suffer from dry skin due to low plasma levels of testosterone and/or DHEA and its sulfate can be treated in accordance with my previous invention.

Menopausal and post-menopausal and other older women usually have a distinct reduction in dehydroepiandrosterone sulfate levels in the blood which is generally accompanied by a reduction in the oil production of the skin and results in dryness of the skin. Such dry skin problems generally affect the entire body. The face and head, however, have the highest population of sebaceous glands and therefore those areas are the most vulnerable to these problems. I previously found that the internal administration of the alcohol or one or more salts of DHEA increases the blood level of DHEA and its sulfate and reduces skin dryness. The reduction of activity of sebaceous glands with aging and at the onset of menopause is reversed by the internal administration of dehydroepiandrosterone alcohol or dehydroepiandrosterone sulfate.

Xeroderma, i.e., dry skin, can be caused by a multiplicity of factors and can be aided by the use of exogenous and water retentive products. I previously found that internal adminstration of dehydroepiandrosterone alcohol or dehydroepiandrosterone sulfate increases the endogenous production and secretion of natural sebum and enhances the water protective barrier of the skin, thus acting as a natural moisturizer.

Oral ingestion or other systemic administration of DHEA or its derivatives results in an increase in oil production by all sebaceous glands over the entire body, an effect that is frequently undesirable or unnecessary. The present invention permits obtaining the above-described desirable effects on a localized basis, only at the places where such effects are desired or necessary.

V. Facchini, Riv. ital. Essenze, Profumi, Piante Officinli, Olii Vegetali - Saponi 41, 69-70, 15.02.1959 describes the effects of natural and synthetic hormones which were known up to the time of the article (September 1958). Tests were performed on persons with dry skin (p.6) but there was no effective way of objective testing for systemic effects of hormone treatments. Facchini could only go by what his patients told him or by visual observation. When Facchini states that after many days of treatment no unwanted effects occurred, he is only referring to visual effects, such as intolerance, irritation and acne, which are specifically mentioned. He could not determine any additional systemic effects which may have been occurring.

The visual check showed signs of invigoration, a better smoothness, softness, increased elasticity and a rosier complexion. These are all cosmetological observations and effects, not medicial effects. At least the invigoration and rosier complexion can be attributed to massage which was the method of application of the hormonal creams to the skin. These visual observations made immediately after treatment were much too soon to see any possible medical effects.

Contrary to what had been expected no particular rehydration of the dehydrated tissues was noticed. It is evident that the amounts of the hormones used by Facchini were insufficient to treat the dry skin except cosmetically. Facchini had evidently expected that rehydration would occur because androgen hormones were known to stimulate sebaceous glands, but such stimulation occured only when the hormones are present in the body systemically in sufficient amounts.

Facchini et al. did not use enough of the hormones to stimulate sebum production to retard and reverse the dryness of the skin and thus, did not observe any unpleasant side effects.

With the present invention substantial systemic effects were found with dehydrotestosterone (DHT) and all other androgens except DHEA. This is a furhter indication that Facchini was not using sufficient amounts of the hormones to produce any medical effects at all. Since Facchini found DHT to produce the most intense action, he should also have observed the bad systemic side effects if he were using amounts sufficient to produce the medical effects obtained with DHEA according to the present invention.

Chemical Abstracts, Volume 99, No. 10, page 370, Ref. No. 76899q (RO-A 79 244) discloses a lotion for controlling acne. The disclosed formulation contains the hormone progesterone, but does not contain DHEA. There is no disclosure of its use for treating dry skin.

Chem. Abstr. 94, 1981, 77274r discloses experiments which test the penetration of various steroids, including DHEA, through the skin. However, there is no disclosure of the use of DHEA to treat dry skin or any other specific skin disease.

EP-A-0 134 954 discloses the use of cholesteryl esters of branched fatty acids for use in accelerating sebum secretion.

FR-A-2 405 069 deals with the treatment of dry skin, but names only testosterone as the preferred androgen. Hence, this reference is contrary to the teachings of the present application.

There have now been discovered compositions for treating dry skin in a patient which involves topically administering to the area of dry skin on the patient a topical vehicle containing an effective amount of DHEA and/or a pharmaceutically acceptable, therapeutically effective derivative thereof. One such derivative of DHEA is the acetate derivative. Other non-limiting derivatives which may be utilized in this invention include valerate, enanthate, and fatty acid ester derivatives.

For patients who have a genetic tendency for acne, the topical compositions of the invention comprise

DHEA and/or a pharmaceutically acceptable, therapeutically effective derivative or analog thereof; a keratolytic agent; and a non-toxic, dermatologically acceptable vehicle. The DHEA and/or derivative thereof is present in an amount which is effective to increase sebum production, while the keratolytic agent is present in an amount sufficient to counteract the formation of acne-like skin lesions without diminishing the effectiveness of DHEA or its derivative.

The preferred keratolytic agents are selected from hydroxybenzoic acids, alpha-hydroxycarboxylic acids and urea. Ortho-hydroxybenzoic acid (salicylic acid) is particularly preferred. The vehicle may be any of a wide variety of preferably non-drying preparations such as tinctures, creams, ointments, gels and lotions.

For the purpose of illustrating the invention, there are provided in the drawings forms which are presently preferred; it being understood, however, that this invention is not limited to the precise arrangements and instrumentalities shown.

Figure 1 is a graph demonstrating the reduction in mean sebum (oil) secretion in males and females in relation to advancing age.

Figure 2 is a graph of dehydroepiandrosterone sulfate levels in the blood as a function of age.

Figure 3 is a pair of bar plots depicting the change in the size of the sebaceous glands of an animal after treatment according to this invention and with various androgens.

Figure 4 is a bar graph comparing DHEA at two different dosages causing equivalent responses as certain doses of testosterone and dihydrotestosterone, respectively.

Figure 5 is a plot of the mean size of the sebaceous gland of the animal ears treated with DHEA in a gel, untreated ears and control animals treated with the gel without DHEA all varying as a function of time.

The sebum excretion rate in animal skin, particularly human skin, may be increased by the topical application of DHEA, either in the form of the free alcohol or in the form of one or more of the analogs or derivatives of DHEA, e.g., the acetate, valerate, enanthate, and fatty acid ester derivatives. As used hereafter in this application unless indicated otherwise, any reference to DHEA will be understood to refer to DHEA and/or derivatives or analogs thereof. The free alcohol form of DHEA is preferred.

The invention further comprises use of dehydroepiandrosterone and/or a pharmaceutically effective derivative thereof for the manufacture of a medical composition for treating dry skin by topical administration thereof, said dehydroepiandrosterone and/or pharmaceutically effective derivative thereof being present in the medicament in an amount of from 0.01 to 1.0% by weight of the composition and sufficient to increase sebum production to an extent sufficient to reduce or retard dryness of the skin, but said amount being such that substantially no undesirable effects are caused locally or systemically.

Typical formulations contain about 1 weight percent DHEA based on the total weight of the formulation, as that amount was found to be effective when topically applied to the area of dry skin to increase the sebum production rate and thereby reduce or retard dryness of the skin. Such topical applications were found to be essentially local in effect with substantially no systemic effect or undesirable side effects.

It has been found, however, that there are concentrations at which DHEA is used in treating dry skin such that the increase in sebum has been accompanied by or associated with the formation of acne-like skin lesions in people who have the genetic tendency for acne. Conventional over-the-counter preparations for treating acne generally act as drying agents and eyfoliators, which are generally irritating to the skin and counterproductive to the general treatment of dry skin. Accordingly, it would be advantageous to have a composition which could counteract the formation of acne-like skin lesions without diminishing the effectiveness of DHEA in improving the sebaceous gland activity of the skin.

For patients who have a genetic tendency for acne, effective formulations may contain about 0.01 to 1.0 weight percent DHEA, typically about 1 weight percent. It has been found that DHEA concentrations on the order of about 0.1 weight percent of the formulations are as effective as the above DHEA formulations in sebum production while preventing the side effect of acne-like skin lesions in certain patients. Concentrations above about 1.0 weight percent DHEA could be used but produce no significant increased effect and are therefore wasteful.

DHEA has been found to be so potent in increasing sebum production that acne is caused in patients who have the genetic tendency to such conditions. That is, the increased sebum was found to form the hardened plugs which clog the pores and cause the formation of cysts which are characteristic of acne and acne-like conditions. The incorporation of a keratolytic agent in the compositions of the present invention was found to substantially prevent the occurence of such conditions.

The keratolytic agents used in the formulations and methods of the present invention may include hydroxybenzoic acids, including ortho, meta or para hydroxybenzoic acids; alpha-hydroxycarboxylic acids, such as glycolic, tartaric, lactic or mandelic acids; or urea. Particularly preferred according to the invention is ortho-hydroxybenzoic acid (salicylic acid), which is a known exfoliator in topical over-the-counter acne treatment preparations, as well as being used in topical preparations for the treatment of warts, corns,

calluses and seborrheic disorders.

It has been reported that alpha-hydroxycarboxyclic acids such as glycolic, tartaric, lactic and mandelic acid have been reported in the literature to be effective keratolytic agents. See e.g., E.J. Van Scott and R.J. Yu, "Substances that Modify the Stratum Corneum by Modulating its Formation", Chapter 10 of Principles of Cosmetics for the Dermatologist, edited by Philip Frost and Stephen N. Horwitz. Moreover, salicylic acid (orthohydroxybenzoic acid) is a known keratolyic agent present in an amount of about 2 percent by weight in over-the-counter preparations such as "PERMOX", "FOSTEX" and "FOSTRIL" (trade marks), used in the treatment of acne. However, these known exfoliators have not to applicant's knowledge been used in preparations to prevent acne formation.

When salicylic acid is used as the keratolytic agent in the formulations of the invention, it is preferably present in the amount of about 0.1 to 2 percent by weight. Other keratolytic agents, which are not as strong as salicyclic acid, may be used in the formulations of the invention in concentrations of about 0.1 to 10 percent by weight of the formulation. The exact concentration of keratolytic agent to be used in the invention will vary somewhat depending upon the particular form of dosage chosen, e.g., ointment, cream, lotion, but may be determined by one of ordinary skill in the art with a minimium of experimentation.

As demonstrated in the specific examples below the compositions of the present invention may contain conventional vehicle ingredients to form lotions, tinctures, creams, gels or ointments which are non-toxic and dermatologically acceptable. In addition to these and other vehicles which will be obvious to those of ordinary skill in the art, it will be understood that the compositions of the present invention may include other ingredients such as hydrocortisone moisturizers, benzoyl peroxide, antibiotics, etc.

To permit quantitative evaluation of the effectiveness and degree of localization of the topical application of various amounts of the free alcohol form of DHEA and its derivatives, e.g., acetate, an animal model (hamsters) was chosen that would permit direct examination and quantitation of the size of sebaceous glands as a function of the treatment according to this invention. The use of a hamster ear for sebaceous gland testing was described by Plewig, G. and Luderschmidt, "Hamster Ear Model for Sebaceous Glands", Journal of Investigative Dermatology, 68: 171-176 (1977).

Hamster sebaceous glands have been used by numerous medical researchers as an effective and medically accepted animal model for the evaluation of pharmacological products and are directly translatable to their use on human subjects. This model has the advantage of reacting to androgens, estrogens, and other hormones and steroid formulations in a manner closely related to those experienced in the treatment of human beings.

Sebaceous gland cells grow in size as they fill with sebum until they reach full maturity at which point they break open and release their sebum to the surface of the skin. Measurement of sebaceous gland size is thus an effective indicator of the amount of sebum (oil) that is delivered to the surface of the skin. Large glands produce large amounts of sebum, while small glands produce small amounts, thus large glands are associated with oily skin; small glands with dry skin. Hamster ears have sebaceous glands which are readily treatable and easily measured. Accordingly, tests were conducted in which one ear of the hamster was treated with DHEA and/or a pharmaceutically acceptable, therapeutically effective derivative thereof in a suitable base vehicle while the contralateral ear (the control) was treated with the same base vehicle without the DHEA, and the resultant changes in the gland size of the ear treated with DHEA and the control animal ear were compared.

When the effect of treatment is systemic, both ears will be affected and significant gland size changes will occur on both ears. It is preferred during testing to have a response at the site of application only, e.g., no systemic effect from local application.

The treatment according to this invention was also compared to other known treatments (use of androgens such as testosterone) to produce sebaceous gland enlargement. These other treatments are generally not usable because of their undesirable side effects and systemic action. In the tests used for evaluation, hamsters were treated on one ear with 1% by weight of the free alcohol form of DHEA in 50 microliters of a suitable vehicle and on the other ear with 50 microliters of the vehicle alone. Similar applications of 1% by weight of various androgens in the same vehicle on other hamsters were used for comparison. A total of 149 hamsters were used to insure statistical validity. Both gel and tincture vehicles were used.

Figure 3 shows a bar plot of the increase in the size of the sebaceous glands of between about 6 and 10 hamsters per group after treatment with the free alcohol form of DHEA and with various other androgens. The outer bar in each instance is the ratio of the mean size of sebaceous glands of the treated hamster ear to the mean size of the sebaceous glands of the ears of control hamsters. The inner bar is the ratio of the mean size of sebaceous glands of the untreated contralateral ear of treated hamsters to the mean size of the sebaceous glands of the ears of control hamsters. The scale is on the left of the plot.

It can be seen from Figure 3, that all the androgens tested produced statistically significant systemic effects as measured by the increase of the gland sizes of the contralateral ear (inner bar), while DHEA had no statistically significant systemic effects. In these tests the hamsters were treated once per day for five days a week for two weeks with 50 microliters of a 1% solution (weight/volume) in a tincture vehicle.

Figure 3 shows the statistically insignificant systemic effect of DHEA compared to the other androgens tested. The curve interconnecting the points in the center of each bar is a plot of the systemic effect as a percent of contralateral untreated ear sebaceous gland size versus the sebaceous gland size of the treated ear. The percentage scale is on the right side of the plot; no systemic effect is at the bottom of the graph corresponding to no increase on the contralateral side and 100% systemic effect is at the top. As is shown, the systemic effect of DHEA was about 15% (statistically insignificant) whereas all the other androgens tested had systemic effects in excess of 50%.

Figure 4 shows a bar graph comparing DHEA at 1% weight/volume in a tincture vehicle to a dose of testosterone ("testo") (0.1%) causing an equivalent response and dehydroepiandrosterone at 5% weight/volume to a dose of dihydrotestosterone ("DHT") (0.1%) causing an approximately equivalent response. Treatment was once per day for five days a week for two weeks. All values represent the ratio of the mean size of sebaceous glands of treated hamsters to the mean size of sebaceous glands of control hamsters. The outer and inner bars are defined as set forth above for Figure 3. The scale is on the left of the plot. Only DHEA showed unilateral performance. The curve (as in Figure 3 and with its scale on the right) shows the systemic effect of DHEA as less than 10% (statistically insignificant), whereas testosterone and dihydrotestosterone have systemic effects of over 80%.

Figure 5 shows a plot of the mean size of sebaceous glands of ears treated once per day for five days a week with DHEA (1% weight/volume) in a gel vehicle, untreated contralateral ears of the same hamsters (designated as contralateral), and control hamsters treated with the gel vehicle without any DHEA (designated as gel), as a function of the time of treatment. The plot shows that two weeks of treatment produced equilibrium and that the effect on the contralateral ears' sebaceous glands was negligible.

The invention will now be described in further detail by reference to the following specific, non-limiting examples.

Examples 1-4

These examples concern the preparation of a tincture, topical cream, topical ointment and topical gel, respectively, using vehicles previously used in other preparations and reformulated to optimize the efficacy of the DHEA. The formulations for these preparations are given in the Table I hereinbelow.

TABLE I

| Dehydropiandrosterone (DHEA) Formulations | | | | | |
|---|---|---|---|---|---|
| Ingredients | | Example No. 1 | Example No. 2 | Example No. 3 | Example No. 4 |
| | | Topical Tincture %w/w | Topical Cream/Lotion %w/w | Topical Ointment %w/w | Topical Gel %w/w |
| 1. | DHEA alcohol acetate valerate, etc any fatty acid ester | 1.0 | 1.0 | 1.0 | 1.0 |
| 2. | Methyl Paraben NF | | .01 | | |
| 3. | Propyl Paraben NF | | .01 | | |
| 4. | Hydroxy Propyl Cellulose (1) | | | | 1.0 |
| 5. | PPG-12-Buteth-16 (2) | | | | 2.0 |
| 6. | Saualane (3) | | 2.0 | | |
| 7. | Glyceryl Monostearate NF | | | 2.0 | |
| 8. | Stearyl Alcohol NF | | 2.8 | | |
| 9. | Cetyl Alcohol NF | | 4.2 | | |
| 10. | Polyethylene Glycol Cetyl Ether (4) | | 5.0 | | |
| 11. | Mineral Oil NF | | 5.0 | | |
| 12. | Butylene Glycol | 4.0 | | 12.0 | 4.0 |
| 13. | Petrolatum USP | | 5.4 | 85.0 | |
| 14. | Alcohol (5) | 89.0 | | | 47.0 |
| 15. | Water | 6.0 | 74.4 | | 45.0 |
| | | 100.0 | 100.0 | 100.0 | 100.0 |

Notes:
(1) available under the trademark KLUCEL from Hercules
(2) available under the trademark UCON fluid 50HB from Union Carbide
(3) available under the trademark ROBANE from Robeco
(4) available under the trademark BRIG 58 from ICI
(5) contains 95% ethanol and 5% water

Example No. 1

Butylene glycol and water were mixed and dissolved into alcohol. The resultant vehicle mixture and DHEA were mixed and dissolved. The resultant formulation was a tincture.

Example No. 2

In this example a topical cream was prepared by first mixing and melting squalane, stearyl alcohol NF, cetyl alcohol, polyethylene glycol cetyl ether, mineral oil NF and petrolatum USP, at 70 degrees C. A second mixture was formed by mixing and dissolving methyl paraben NF and propyl paraben NF in water, at 70 degrees C. The second mixture was slowly added to and mixed with the first mixture to form an emulsion. DHEA was dispersed in the resultant emulsion at 50 degrees C. The resultant composition was slowly cooled with mixing until the composition reached room temperature.

Example No. 3

In this example a topical ointment was prepared. As a first step, glyceryl monostearate was mixed and melted in petrolatum USP at 70 degrees C. As a second step, DHEA was mixed and dissolved in butylene glycol at 70 degrees C. The resultant composition of step 2 was slowly added to the resultant composition of step 1, with mixing. This mixture was then cooled to its congealing point with mixing and then cooled to room temperature without mixing.

Example No. 4

In this example a topical gel was prepared. As a first step, hydroxy propyl cellulose was hydrated and dissolved into water. As a second step, DHEA, butylene glycol and PPG-12-Buteth-16 was dissolved in alcohol. Slowly the resultant mixture of step 2 was added into the resultant mixture of step 1 with mixing until a gel formed.

Examples 5-8

The following specific, non-limiting examples concern the preparation of a topical lotion, topical cream, topical ointment and topical gel, respectively, using vehicles previously used in other preparations and reformulated to optimize the efficacy of the DHEA and keratolytic agent. The formulations for these preparations are Given in Table II hereinbelow.

Example No. 5

Propylene glycol and water were mixed and dissolved into alcohol. The resultant vehicle mixture, salicylic acid and DHEA were mixed and dissolved. The resultant formulation was a topical lotion or tincture.

Example No. 6

In this example a topical cream was prepared by first mixing and melting propylparaben, stearic acid, cetyl alcohol, glyceryl monostearate, lanolin oil, mineral oil and sesame oil at 70 degrees C. A second mixture was formed by mixing and dissolving methylparaben, triethanolamine and propylene glycol in water at 70 degrees C. The second mixture was slowly added to and mixed with the first mixture to form an emulsion. DHEA, salicylic acid and Carbomer 940 were dispersed in the resultant emulsion at 50 degrees C. The resultant composition was slowly cooled with mixing until the composition reached room temperature.

Example No. 7

In this example a topical ointment was prepared. As a first step, glyceryl monostearate was mixed and melted in petrolatum at 70 degrees C. As a second step, DHEA and salicylic acid were mixed and dissolved in propylene glycol at 70 degrees C. The resultant composition of step 2 was slowly added to the resultant composition of step 1, with mixing. This mixture was then cooled to room temperature without mixing.

Example No. 8

In this example a topical gel was prepared. As a first step, hydroxy propyl cellulose was hydrated and dissolved into water. As a second step, DHEA, salicylc acid, butylene glycol and PPG-12-Buteth-16 were dissolved in alcohol. Slowly the resultant mixture of step 2 was added into the resultant mixture of step 1 with mixing until a gel formed.

TABLE II

DHEA - Salicylic Acid Formulations

| | Example No. 5 Topical Lotion %w/w | Example No. 6 Topical Cream %w/w | Example No. 7 Topical Oint. %w/w | Example No. 8 Topical Gel %w/w |
|---|---|---|---|---|
| Ingredients | | | | |
| Actives | | | | |
| DHEA (free alcohol) | 1.0 | 1.0 | 1.0 | 1.0 |
| Salicylic acid | 1.0 | 1.0 | 1.0 | 1.0 |
| Inactives | | | | |
| Methylparaben | | 0.1 | | |
| Propylparaben | | 0.1 | 0.1 | |
| Hydroxy propyl cellulose (1) | | | | 1.0 |
| PPG-12-Buteth-16 (2) | | | | 2.0 |
| Lanolin oil | | 5.0 | | |
| Mineral oil | | 4.0 | | |
| Sesame oil | | 4.0 | | |
| Cetyl alcohol | | 5.0 | | |
| Glyceryl monostearate | | 2.0 | 3.0 | |
| Stearic acid | | 2.0 | | |
| Triethanolamine | | 1.0 | | 0.2 |
| Propylene glycol | 5.0 | 5.0 | 12.0 | 5.0 |
| Alcohol (3) | 87.0 | | | 45.0 |
| Carbomer 940 (4) | | 0.1 | | 0.2 |
| Petrolatum | | | 82.9 | |
| Water | 6.0 | 69.7 | | 42.4 |
| | 100.0 | 100.0 | 100.0 | 100.0 |

Notes:
(1) available under the trademark KLUCEL from Hercules
(2) available under the trademark UCON fluid 50 HB from Union Carbide
(3) contains 95% ethanol and 5% water
(4) available under the trademark CARBOPOL 940 from B.F. Goodrich

## Claims

1. Use of dehydroepiandrosterone and/or a pharmaceutically effective derivative thereof for the manufacture of a medical composition for treating dry skin by topical administration thereof, said dehydroepiandrosterone and/or pharmaceutically effective derivative thereof being present in the medicament in an amount of from 0.01 to 1.0% by weight of the composition and sufficient to increase sebum production to an extent sufficient to reduce or retard dryness of the skin, but said amount being such that substantially no undesirable effects are caused locally or systemically.

2. Use of a composition according to claim 1 comprising further a non-toxic dermatologically acceptable vehicle; and 0.1 to 10.0% by weight of the composition of a keratolytic agent, which counteracts the formation of the acne-like skin lesions without diminishing the effectiveness of dehydroepiandrosterone

or its derivative.

3. Use according to claim 2 for manufacturing of a medicament for the treatment of a patient with a genetic tendency for acne.

4. Use according to any of claims 1-3 of the composition for the manufacturing of a medicament in the form of a tincture, a cream, an ointment or a gel.

5. Use according to claim 2 wherein the keratolytic agent is selected from hydroxybenzoic acids, alpha-hydroxycarboxylic acids and urea.

6. Use according to claim 5 wherein the alpha-hydroxycarboxylic acid is selected from glyolic, tartaric, lactic and mandelic acids.

7. Use according to claim 5 wherein the keratolytic agent is salicylic acid.

8. Use according to claim 7 wherein the salicylic acid is present in an amount of 0.1 to 2% by weight of the composition.

9. Use according to claim 2 wherein dehydroepiandrosterone free alcohol is present in an amount of 1.0% by weight and the keratolytic agent is salicylic acid present in an amount of 1.0% by weight of the composition.

10. Use according to claim 1 wherein the dehydroepiandrosterone is in the form of the free alcohol or a derivative selected from acetate, enanthate, valerate and fatty acid esters.

**Patentansprüche**

1. Verwendung von Dehydroepiandrosteron und/oder eines pharmazeutisch wirksamen Derivates davon, für die Herstellung eines Arzneimittels, zur Behandlung trockener Haut mittels topischer Verabreichung, wobei Dehydroepiandrosteron und/oder das pharmazeutisch wirksame Derivat davon in dem Medikament in einer Menge von 0.01 bis 1.0 Gewichtsprozent der Zusammensetzung vorhanden sind, und ausreicht, um die Talgproduktion auf ein Maß zu erhöhen, das ausreicht, die Trockenheit der Haut zu vermindern oder zu verzögern, die Menge aber derart ist, daß im wesentlichen keine unerwünschten lokalen oder systemischen Effekte verursacht werden.

2. Verwendung einer Zusammensetzung gemäß Anspruch 1, die ferner einen nicht-toxischen, dermatologisch-akzeptablen Träger, und einen keratolytischen Wirkstoff in 0.1 bis 10.0 Gewichtsprozent der Zusammensetzung aufweist, welcher der Bildung von akneähnlichen Hautveränderungen entgegenwirkt, ohne die Wirksamkeit des Dehydroepiandrosterons oder seines Derivates zu vermindern.

3. Verwendung gemäß Anspruch 2 zur Herstellung eines Medikamentes für die Behandlung eines Patienten mit einer genetischen Neigung zur Akne.

4. Verwendung der Zusammensetzung gemäß einem der Ansprüche 1-3 für die Herstellung eines Medikamentes in der Form einer Tinktur, einer Creme, einer Salbe oder eines Gels.

5. Verwendung gemäß Anspruch 2, in welchem der keratolytische Wirkstoff ausgewählt ist aus Hydroxybenzoesäuren, alpha-Hydroxycarbonsäuren und Harnstoff.

6. Verwendung gemäß Anspruch 5, in welchem die Alpha-Hydroxycarbonsäure ausgewählt ist aus Glykol-, Wein-, Milch- und Mandelsäuren.

7. Verwendung gemäß Anspruch 5, in welchem der keratolytische Wirkstoff Salicylsäure ist.

8. Verwendung gemäß Anspruch 7, in welchem die Salicylsäure in einer Menge von 0.1 bis 2 Gewichtsprozent der Zusammensetzung vorliegt.

9. Verwendung gemäß Anspruch 2, in welchem Dehydroepiandrosteron als freier Alkohol in einer Menge von 1.0 Gewichtsprozent vorliegt, und der keratolytische Wirkstoff Salicylsäure ist, die in einer Menge von 1.0 Gewichtsprozent der Zusammensetzung vorliegt.

10. Verwendung gemäß Anspruch 1, in welchem das Dehydroepiandrosteron in der Form des freien Alkohols oder eines Derivates, ausgewählt aus Essigsäure-, Önanthsäure-, Valeriansäure- und Fettsäureestern, vorliegt.

**Revendications**

1. Utilisation de déshydroépiandrostérone et/ou de l'un de ses dérivés pharmaceutiquement efficace pour la fabrication d'une composition médicale pour le traitement de la sécheresse de la peau par administration topique de celle-ci, ladite déshydroépiandrostérone et/ou son dérivé pharmaceutiquement efficace étant présents dans le médicament suivant une quantité comprise entre 0,01 et 1,0 % en poids de la composition et suffisante pour augmenter la production de sébum dans une mesure suffisante pour réduire ou retarder la sécheresse de la peau, mais ladite quantité étant telle que pratiquement aucun effet indésirable ne sera produit localement ou systémiquement.

2. Utilisation d'une composition selon la revendication 1, comprenant en outre un véhicule non toxique acceptable dermatologiquement; et de 0,1 à 10,0 % en poids de la composition d'un agent kératolytique, qui contrecarre la formation dans la peau de lésions semblables à l'acné sans diminuer l'efficacité de la déshydroépiandrostérone ou de son dérivé.

3. Utilisation selon la revendication 2 pour la fabrication d'un médicament pour le traitement d'un malade ayant une tendance génétique à l'acné.

4. Utilisation selon l'une quelconque des revendications 1 à 3 de la composition pour la fabrication d'un médicament sous la forme d'une teinture, d'une crème, d'un pommade ou d'un gel.

5. Utilisation selon la revendication 2, dans laquelle l'agent kératolytique est choisi parmi les acides hydroxybenzoïques, les acides alpha-hydroxycarboxyliques et l'urée.

6. Utilisation selon la revendication 5, dans laquelle l'acide alpha-hydroxycarboxylique est choisi parmi les acides glyolique, tartrique, lactique et mandélique.

7. Utilisation selon la revendication 5, dans laquelle l'agent kératolytique est l'acide salicylique.

8. Utilisation selon la revendication 7, dans laquelle l'acide salicylique est présent suivant une quantité comprise entre 0,1 et 2 % en poids de la composition.

9. Utilisation selon la revendication 2, dans laquelle de l'alcool libre de déshydroépiandrostérone est présent suivant une quantité de 1,0 % en poids et l'agent kératolytique est l'acide salicylique présent dans une quantité de 1,0 % en poids de la composition.

10. Utilisation selon la revendication 1, dans laquelle la déshydroépiandrostérone est sous la forme de l'alcool libre ou d'un dérivé choisi parmi l'acétate, l'énanthate, le valérate et les esters d'acides gras.

FIG. 1

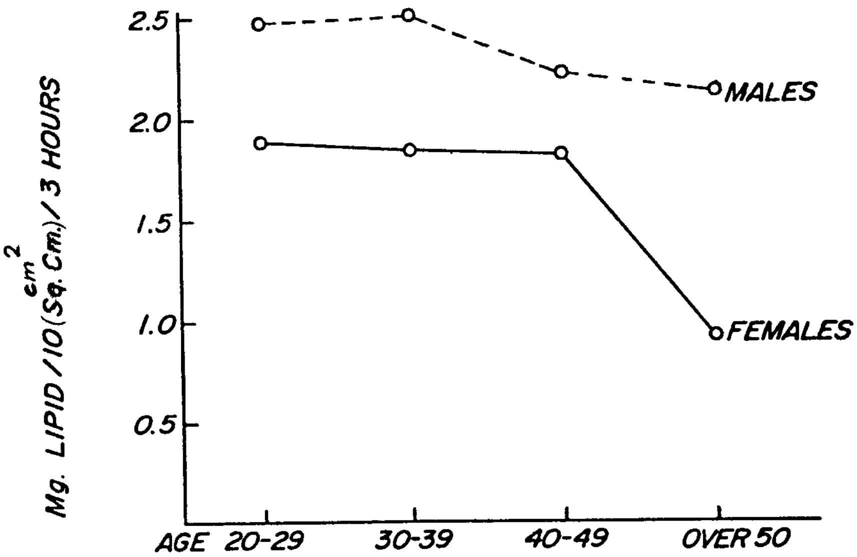
2.5
2.0
1.5
1.0
0.5
Mg. LIPID / 10 (Sq. Cm.) / 3 HOURS
MALES
FEMALES
AGE 20-29
30-39
40-49
OVER 50

FIG. 2

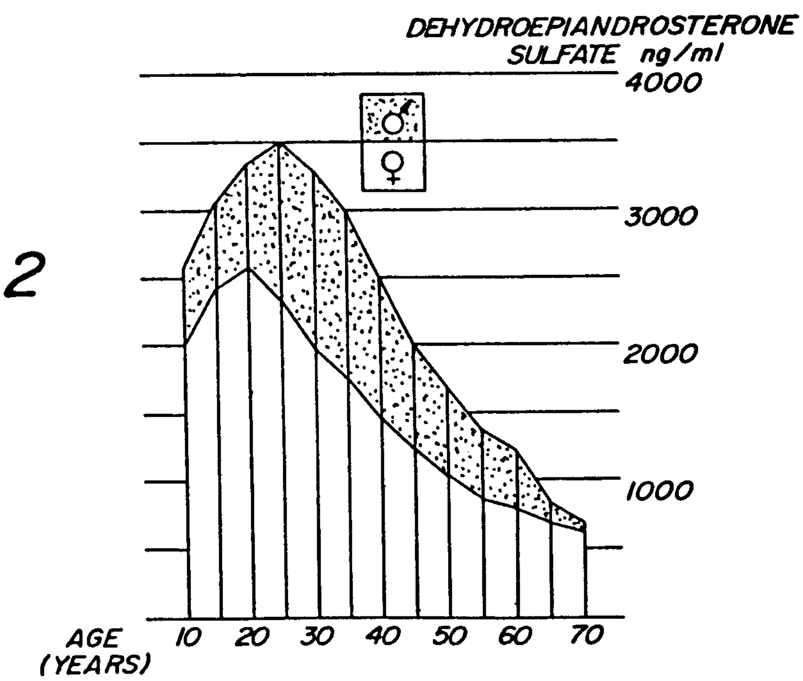
DEHYDROEPIANDROSTERONE SULFATE ng/ml
4000
3000
2000
1000
♂
♀
AGE (YEARS)
10
20
30
40
50
60
70

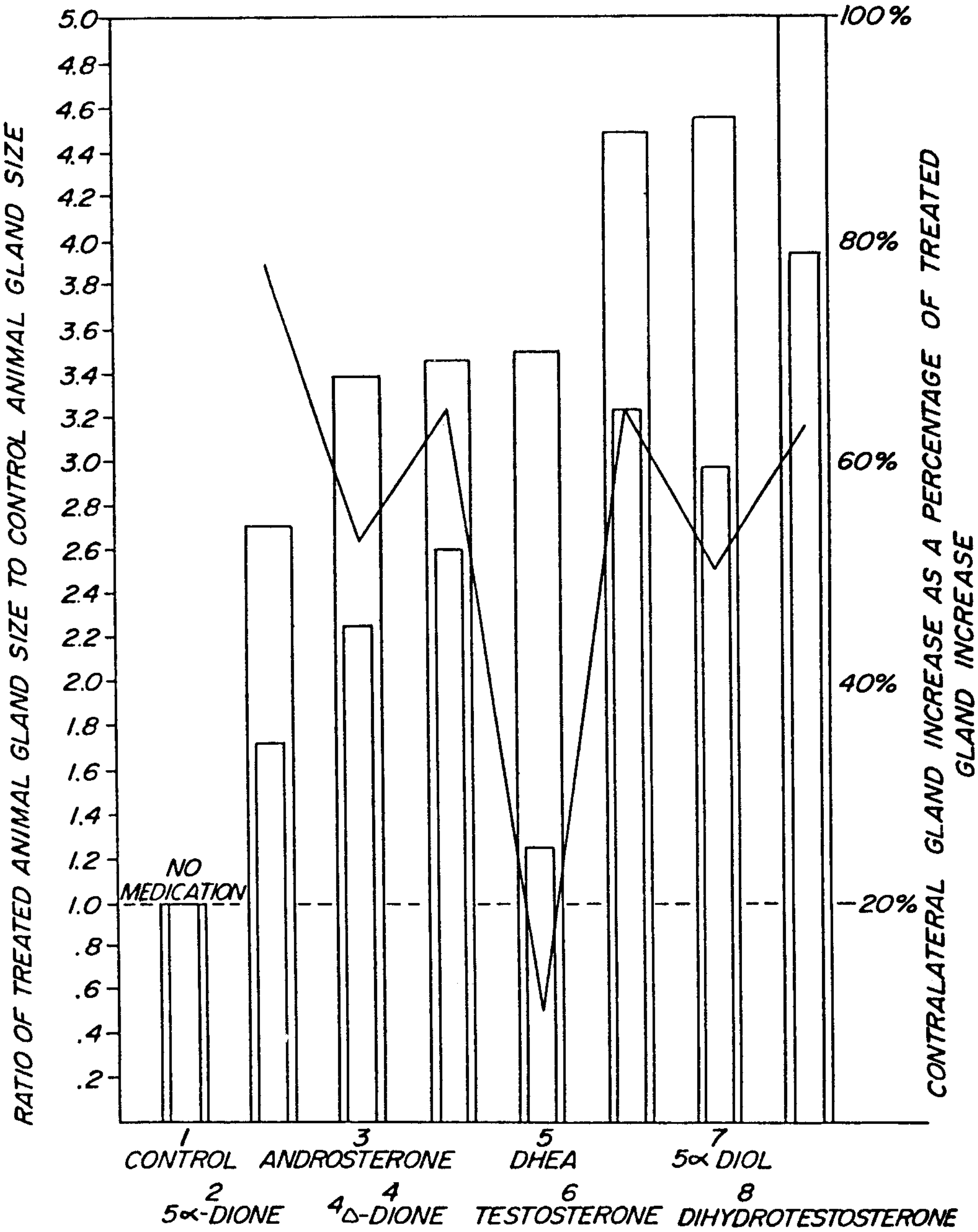


MEAN RATIO OF TREATED TO CONTROL EAR SEBACEOUS GLAND SIZES FOR VARIOUS MEDICATIONS

FIG. 3

FIG. 4

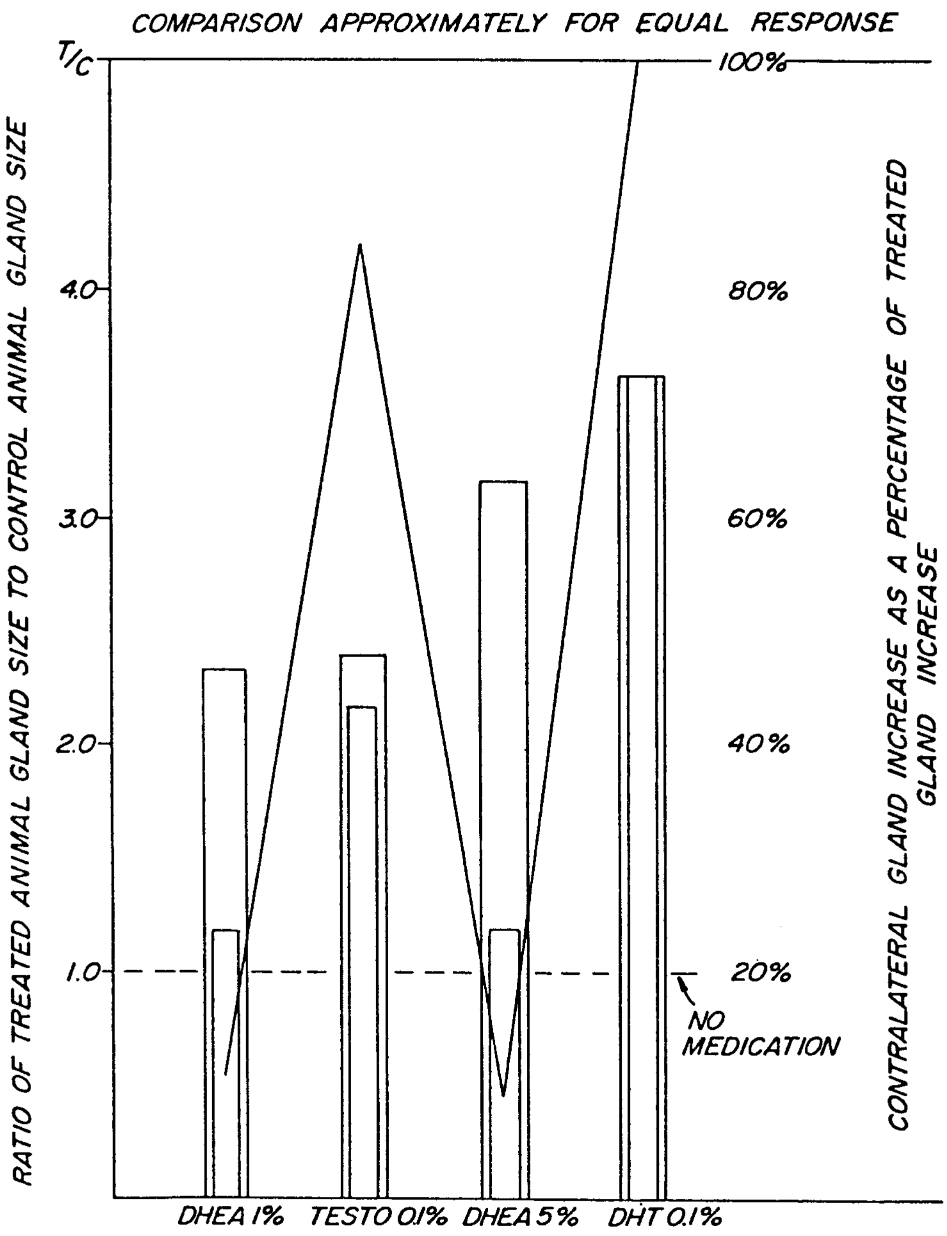


COMPARISON FOR EQUIVALENTLY EFFECTIVE LEVELS OF DEHYDROEPIANDROSTERONE (DHEA) V. TESTOSTERONE (TESTO) AND DIHYDROTESTOSTERONE (DHT)

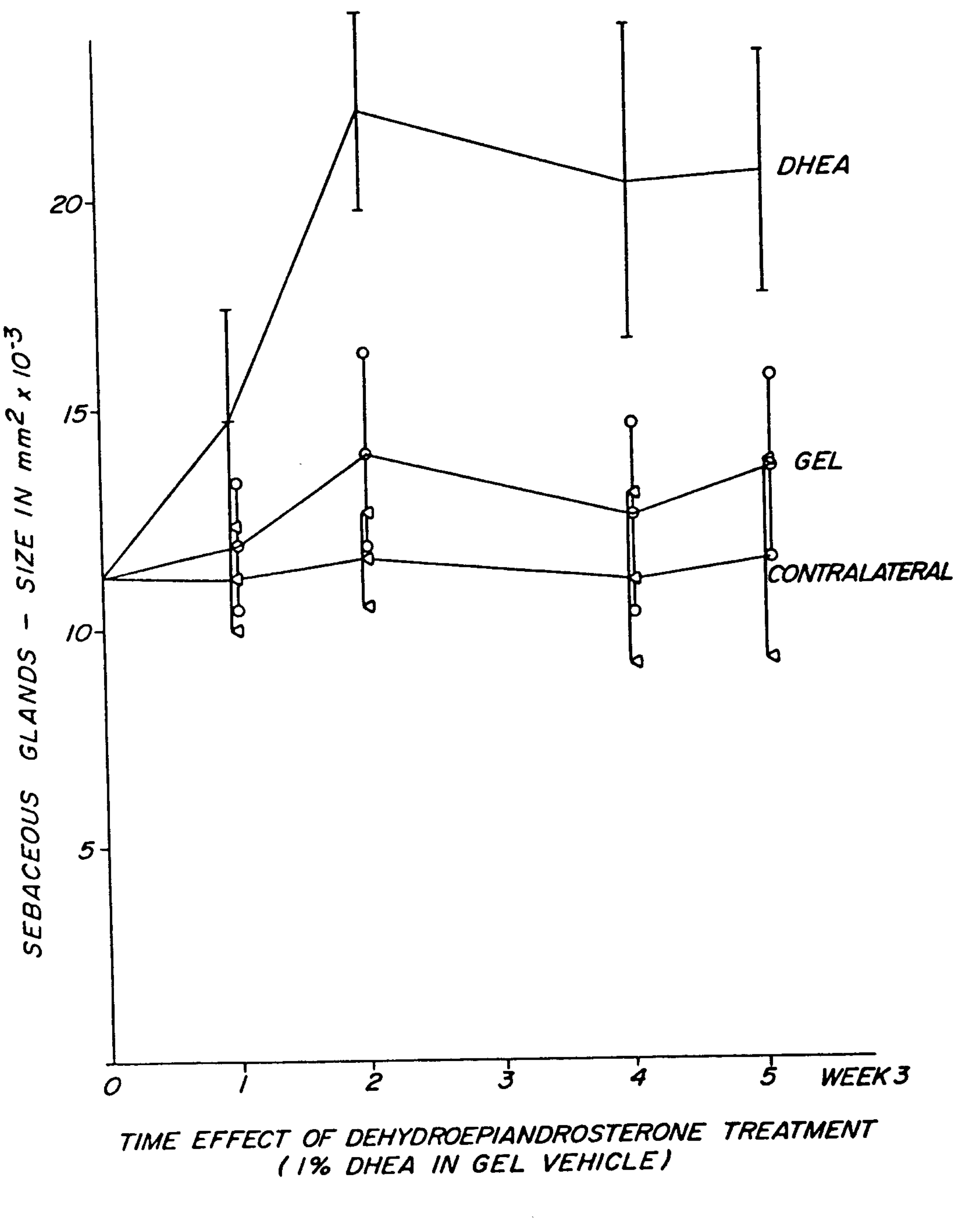

DHEA
GEL
CONTRALATERAL
20
15
10
5
SEBACEOUS GLANDS - SIZE IN $mm^2 \times 10^{-3}$
0
1
2
3
4
5
WEEKS
TIME EFFECT OF DEHYDROEPIANDROSTERONE TREATMENT
(1% DHEA IN GEL VEHICLE)


FIG. 5